# EUROPEAN PATENT APPLICATION

(11) **EP 1 712 908 A2**
(43) Date of publication of application: **18.10.2006**
(21) Application number: 06112467.3
(22) Date of filing: 11.04.2006
(51) Int. Cl.: G01N 33/00

(54) **Sensor arrangement and method for the qualitative and quantitative detection of chemical substances and/or mixtures of substances in an environment**

(30) Priority: 11.04.2005 IT TO20050239
(71) Applicant: Consorzio Nanowave, 16153 Genova (IT)
(72) Inventor: Ridi, Andrea, I-16100 Genova (IT); Muselli, Marco, I-16149 Genova (IT)
(74) Representative: Deambrogi, Edgardo

(57) **Abstract**

What is described is a sensor arrangement of the electronic nose type and a method for the qualitative and quantitative detection of chemical substances and/or mixtures of substances in an environment. The sensor arrangement comprises an array of sensors (10), capable of emitting a set of response signals correlated with the presence and/or concentration of at least one chemical substance, and an electronic processing and recognition system including a Boolean learning machine (34), arranged to classify the response signals generated by the sensor array (10) by the application of at least one predetermined collection of binary classification rules, adapted to discriminate between a pair of predetermined complementary outcomes of the detection.

## Description

The present invention relates to a sensor arrangement and a method for the qualitative and quantitative detection of chemical substances and/or mixtures of substances in an environment.

More specifically, the invention relates to a sensor arrangement comprising:
an array of sensors, each of which is capable of modifying at least one of its own physical parameters with respect to a reference condition in the presence of at least one predetermined chemical substance, and emitting a corresponding electrical response signal indicating the extent of modification of the said parameter,
the set of the response signals of the sensor array being correlated with the presence and/or concentration of the said at least one substance; and
electronic processing and recognition means of the automatic learning type, arranged to receive the said response signals at their input and to detect the presence and/or concentration of at least one chemical substance being searched for, on the basis of a set of training data acquired in a learning phase.

The invention also relates to a method for detecting substances and/or mixtures by means of a sensor arrangement of the type specified above.

The detection of the presence of specific substances in mixtures, including complex mixtures, and the estimation of their concentration forms the basis of the process of analysing and identifying odours in an environment by means of artificial olfactory sensing systems.

Such a system includes an array of partially selective sensors, known as an "electronic nose", capable of recognizing simple and complex odours, in which each sensor is sensitive to one or more chemical substances. A substance or mixture of substances coming into contact with the matrix of sensors stimulates in each of the sensors a variation of one or more physical or chemical parameters with respect to a reference condition.

There are many values which can be measured, for example the shifting of the resonant frequency of a quartz crystal or the variations of electrical resistance or impedance of a film of chemically sensitive and intrinsically conductive material, deposited on an insulating substrate and electrically connected to a pair of conducting terminals or electrodes.

Planar sensors of the sensitive film type are preferably used because of their greater sensitivity, their electrical signals sent in response to predetermined chemical substances being correlated with each other and analysed by "pattern recognition" methods in order to classify the odours and quantify their concentration.

In the known art, the response signals of the sensors are interpreted by processing systems of the automatic learning type, such as, for example, neural networks. An example of an apparatus and method of this type for estimating the concentration of odours by means of an electronic nose are described in US Patent 6 411 905.

Unfortunately, conventional sensor systems produced by using such neural networks are complicated and expensive.

To overcome this drawback and to enable these systems to be used in apparatus and devices intended for use by large numbers of consumers, and not only in expensive industrial facilities or research laboratories, it would be useful for the sensor array forming the electronic nose to be combined with an inexpensive processing electronics.

However, since a neural network is typically trained on computers, it fails to interpret the sensor responses correctly when it is subsequently implemented in a simple microcontroller or in inexpensive electronic systems in general, since the fundamental algorithms of the neural network itself diverge because the calculation accuracy differs from that of the training phase on computers.

The object of the invention is to provide a technology for providing an artificial olfactory sensor system at low cost, in order to make the use of such a system convenient even in apparatus and devices for mass consumption, by overcoming the drawbacks of the known art.

For this purpose, the invention proposes a sensor arrangement of the electronic nose type, characterized in that the processing means include a Boolean learning machine, arranged to carry out classification of the set of response signals generated by the sensor array by applying at least one predetermined collection of binary classification rules, adapted to discriminate one outcome in a pair of predetermined complementary outcomes of the detection.

The invention also proposes a method for the qualitative and quantitative detection of chemical substances and/or mixtures of substances in an environment, characterized in that it comprises the operations of:
- generating, in a learning phase and on the basis of a set of training response signals, at least one predetermined collection of binary classification rules adapted to perform a discrimination in a pair of predetermined complementary outcomes of the detection;
- configuring the processing means in accordance with the said collection of rules; and
- classifying the set of response signals generated by the sensor array by application of the said at least one predetermined collection of rules.

Specific embodiments of the invention are defined in the dependent claims.

To summarize, the invention is based on the association of the sensor array with a Boolean learning machine, particularly one trained with the known Hamming Clustering (HC) algorithm, by means of which the response signals of the sensors forming the electronic nose array are advantageously processed by the Boolean learning machine by the application of a predetermined collection of binary classification rules, generated by application of the Hamming Clustering algorithm to a set of training data.

The Hamming Clustering algorithm for generating binary classification rules is described by M. Muselli and D. Liberati in the article *"*Training Digital Circuits with Hamming Clustering", published in IEEE Transaction on Circuits and Systems - I: Fundamental Theory and Applications, vol. 47, no. 4, April 2000, pp. 513-527, and in the article *"*Binary Rule Generation via Hamming Clustering", published in IEEE Transaction on Knowledge and Data Engineering, vol. 14, no. 6, November/December 2000, pp. 1258-1268, the contents of which should be consulted for information on the general operating principles.

Since the classification rules which are adopted are of the Boolean type, they can be implemented by means of combinatorial logic networks, simple microcontrollers or any other inexpensive device capable of evaluating Boolean expressions, without problems of approximation and convergence of the algorithms between the training phase and the subsequent construction of the machine.

A collection of binary classification rules makes it possible to discriminate between a pair of predetermined complementary outcomes of the detection, representing for example the presence or absence of a predetermined chemical substance in the environment, or providing an indication of the comparison of the concentration of a substance being searched for with a predetermined threshold value.

A plurality of binary classification rules can be used to discriminate among a plurality of predetermined outcomes of the detection, for example those representing corresponding concentrations of a chemical substance in a mixture, in order to provide a classification indicating a quantitative measurement of the concentration of the said substance.

The learning machine can advantageously be arranged for the successive application of collections of rules adapted to detect different chemical substances, so as to provide a sensor arrangement of the programmable type which can be specialized for the recognition of different substances by automatically varying the collection of rules according to the substance being searched for.

The preferred method for generating a collection of rules includes the operations of:
encoding each training response signal sent by the sensor array as a corresponding binary string by application of a predetermined transformation functions capable of preserving sequencing and distance properties of the values which each signal can take;
concatenating the binary strings generated by each encoding operation and associating them with an outcome of the detection; and
synthesizing the expression of a Boolean function with AND/OR operators of the encoded signals, adapted to discriminate between a pair of predetermined outcomes of the detection.

The encoding of each response signal as a binary string includes the discretization of the value of the signal and the association of a binary string with each discrete value that can be taken by the signal, according to "thermometer encoding".

The expression of the Boolean function is synthesized by application of the Hamming Clustering algorithm and includes the aggregation of binary strings which are associated with a single outcome of the detection and which are close in terms of the Hamming distances, so as to generate prime implicants of a Boolean function expression in the form of a sum of logical products.

Advantageously, each logical product of the Boolean expression forming the basis of a collection of rules of the learning machine is intrinsically translatable into an intelligible rule of the "if-then" type.

Further characteristics and embodiments of the invention will be disclosed more fully in the following detailed description provided by way of example and without restrictive intent, with reference to the attached drawings, in which:
Figure 1 is a block diagram showing an example of a sensor arrangement proposed by the invention; and
Figure 2 is a partial perspective view of a sensor array of the arrangement of Figure 1.

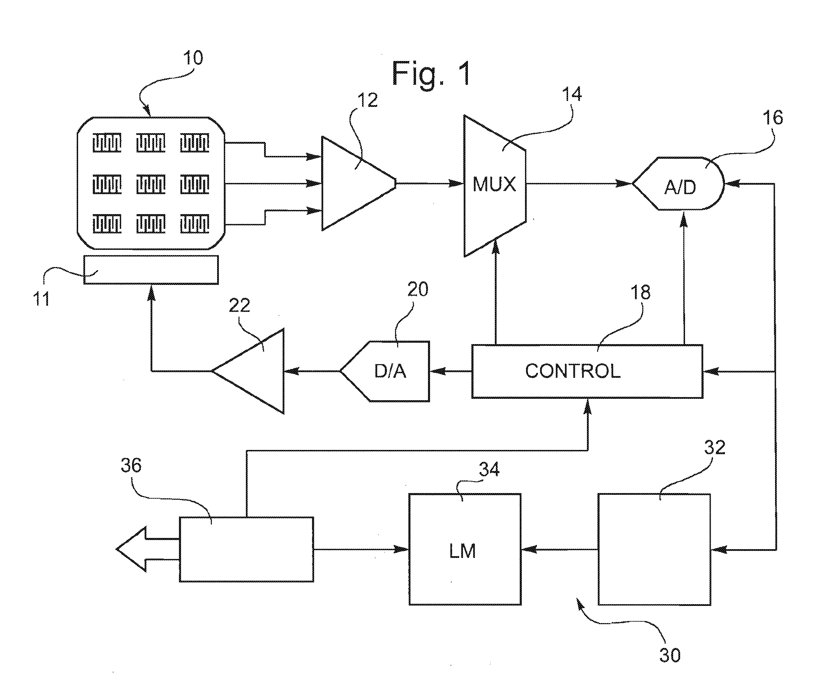

A preferred embodiment of the sensor arrangement proposed by the invention is shown in Figure 1.

The number 10 indicates an array of sensors, each of which is capable of modifying at least one of its own physical or chemical parameters with respect to a reference condition in the presence of at least one predetermined chemical substance, and emitting a corresponding electrical signal indicating the extent of modification of the said parameter.

Preferably, the array consists of a plurality of nanostructured sensors of the thin-film type, comprising for example a nanostructured film of composite material such as TiO₂ o SnO₂, generally deposited on an insulating substrate such as alumina, which forms a chemically sensitive resistive element interposed between a pair of conducting terminals or interdigitated electrodes (made from gold, for example).

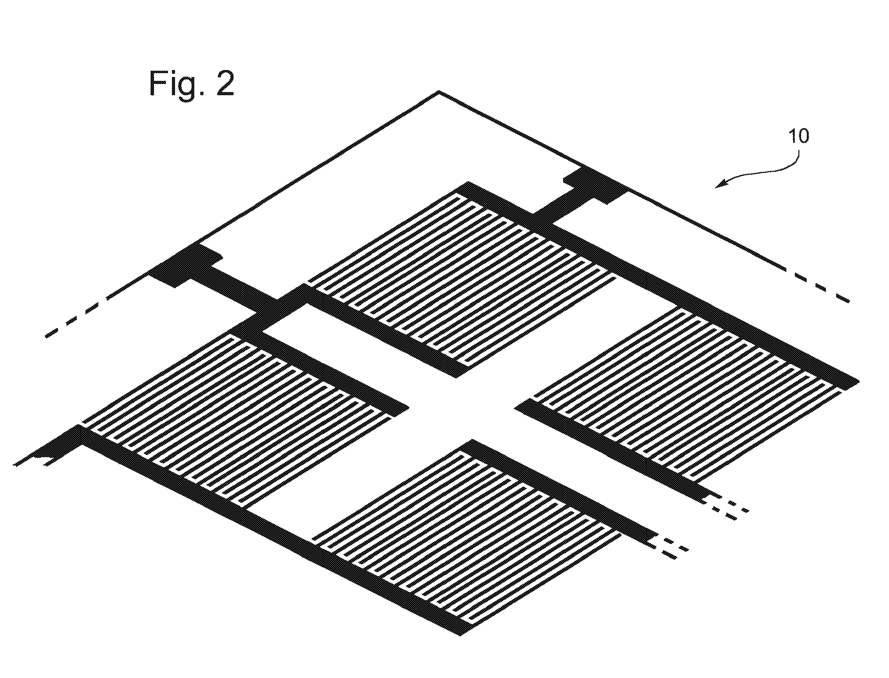

Figure 2 shows by way of example a portion of a sensor array of the type used in the arrangement proposed by the invention, comprising four sensors and corresponding connecting tracks.

The sensors are produced, for example, by depositing the nanostructured film on corresponding pads of the insulating substrate. Platinum heating devices 11, configured as screen printed resistive lines (not shown) with a sinusoidal layout, deposited on the opposite side of the substrate from each pad, enable the sensors to be brought to their operating temperature (approximately 300°C) and held there. The sensors differ from each other in respect of the sensitive film deposition parameters, but have the same chemical composition.

The response signals emitted by each sensor of the array are supplied to the input of a data acquisition system comprising a decoupling impedance matching unit 12, an analogue multiplexer 14 and an analogue-digital converter circuit 16.

The impedance matching unit 12 is provided because the sensors used have a rather high equivalent resistance, of the order of approximately 600 MQ - 1GΩ. It is constructed in such a way as to improve the signal/noise ratio at the input of the multiplexer 14.

The multiplexer 14 has the function of sequentially routing all the signals generated by the sensors of the array towards the analogue/digital converter 16 located downstream.

The output of the analogue/digital converter 16 is connected to a control module 18, preferably implemented in a microcontroller, designed to operate the multiplexer 14, to control the dynamics of the A/D converter 16 and to control the heating devices 11. The module 18 is connected to the heating devices through a digital/analogue converter 20 and a power amplifier unit 22 capable of supplying the power required to keep the temperature of the sensitive film of the sensors constant during the measurement time, using a control feedback circuit if necessary.

The output of the A/D converter 16 is also connected to means for processing the response signals of the sensor array, indicated as a whole by the number 30 in the figure.

The processing means 30 include a module 32 for extracting characteristics of the response signals, and a Boolean learning machine 34 adapted to classify the aforesaid signals on the basis of a predetermined collection of binary classification rules, as described more fully in the remainder of the description.

In a preferred embodiment, the sensor arrangement proposed by the invention also includes a module 36 for interfacing with a communication network for connection to remote programming stations. The communication interface module 36 is connected to the control module 18 for the transfer of control signals of the circuit for data acquisition from the sensors, and to the Boolean machine 34 for its remote programming.

When exposed to a predetermined chemical substance or mixture of substances, each sensor of the array 10 of the preferred type described above varies its resistance characteristics with respect to the resistance in pure air, and supplies a corresponding electrical signal indicating this modification, which forms a component of a response signal vector, known more briefly as a response vector, of the whole array 10.

The set of signals appropriately processed by the acquisition circuit 12-16 is supplied to the characteristics extraction module 32, which can rapidly acquire the value of each signal in a transient phase of the variation of the resistive characteristics of the sensors, and can estimate by calculation a steady-state value of the signal according to predetermined rules.

The learning machine 34 comprises, in the preferred embodiment, a programmable logic device adapted to store a predetermined collection of rules for the binary classification of the response vectors acquired at the input.

The aforesaid programmable logic device can be a microcontroller, for example an 8-bit microcontroller, in which the collection of classification rules can be implemented by means of a sequence of logical rules of the "if-then" or "if-then-else if" type.

This collection of rules corresponds to the expression of a two-level Boolean function in the form of a sum of logical products of binary variables, each indicating the comparison between a signal of the response vector and a corresponding predetermined threshold value, in other words the variation of the resistance of the corresponding sensor with respect to the resistance in pure air.

Alternatively, the learning machine 34 can comprise a combination of logic gates configured as a combinatorial logic network, for example a combination of variable-configuration logic gates.

The collection of rules is obtained by the Boolean learning machine in a learning phase on the basis of a set of training data, using a method for generating binary classification rules, preferably based on the application of the known Hamming Clustering algorithm.

In detail, the learning machine is supplied, in a training phase, with a set of training response vectors, representing the presence and/or concentration of known chemical substances and/or mixtures of substances. In the learning phase, the sensors are characterized in the presence of different gas mixtures. Since all the sensors generally respond to the gases in the mixture, it is necessary to produce a learning machine which makes it possible to discriminate efficiently between very similar gases by correlating the responses received from all the sensors.

The components of each training response vector are encoded as corresponding binary strings by the application of a predetermined transformation function capable of preserving the sequencing and distance properties of the values which each of them can take. The binary strings generated in this way are concatenated and associated with an outcome of the detection, in other words with a binary value representing the presence or absence of a predetermined chemical substance being searched for, or representing the outcome of the comparison of the concentration of a substance in a mixture with a predetermined threshold value.

Preferably, the encoding of the components of each response vector as binary strings includes the discretization of the value of each component of the vector and the association of a binary string with each discrete value, by "thermometer encoding".

Finally, the expression of a Boolean function of the said encoded components is synthesized, which is adapted to discriminate between a pair of predetermined outcomes of the detection.

Preferably, the Boolean function expression is a sum of logical products, and is therefore an expression of the logical operators AND and OR only.

The Boolean function expression is synthesized by application of the Hamming Clustering algorithm, which is described in the literature cited above and is therefore not discussed in detail here. Briefly, this algorithm aggregates the binary strings, generated by the conversion of the values of the components of the response vector from real number space to binary number space, which are associated with a single outcome of the detection and which are close in terms of the Hamming distance. This makes it possible to generate prime implicants of a Boolean function expression in the form of a sum of logical products.

The method proposed by the invention is described below with reference to a practical example.

In the application described by way of example, a series of mixtures having characteristics similar to those which may be encountered in the typical conditions of use of a sensor for detecting ethanol, propanol and methanol were defined.

The response of the individual sensors in the presence of different mixtures of ethanol, propanol and methanol was then measured, and this was followed by the generation of rules for binary classification which discriminate between a pair of predetermined complementary outcomes of the detection, for example between the case in which ethanol is present with a concentration of less than 30 ppm and the case in which it is present with a concentration of more than 30 ppm, regardless of the concentration of the other gases present in the mixture.

The learning machine trained in this way generated a collection of classification rules which could distinguish between two discrete cases, corresponding to the two possible outcomes of the detection, namely: a first outcome, indicated by "0" in the example, in which ethanol was present in a concentration of less than 30 ppm, and a second outcome, indicated by "1", in which ethanol was present in a concentration of more than 30 ppm.

More precisely, the learning machine generated two subsets of rules, namely the rules describing all the cases with the outcome "1" and some with the outcome "0", and the rules describing all the cases with the outcome "0" and some with the outcome "1".

In the specific example, the collection of rules generated is that reported below, in which for each rule there is an indication of its relevance, in other words the percentage of the cases which this rule can describe within the set of training data, and the conditions in terms of the resistances of groups of sensors in the array. In particular, the values shown below as R4, R6, R7 and R10 indicate the variations of resistance of the sensitive films of the corresponding sensors when exposed to the test mixtures.
1. Rule 1, with a relevance of 0.447 (based on 400 examples) and an error of 0.000 (based on 600 examples)
   Outcome "0", no. of conditions --> 2
   Condition 1 (relevance 0.680000): R4 <= 13.3400
   Condition 2 (relevance 0.141667): R7 > 7.7150
   Stat.: right --> 400,0.4475; wrong --> 600,0.0000
2. Rule 2, with a relevance of 0.425 (based on 400 examples) and an error of 0.000 (based on 600 examples)
   Outcome "0", no. of conditions --> 2
   Condition 1 (relevance 0.790000): R4 <= 12.2950
   Condition 2 (relevance 0.078333): R7 > 6.7250
   Stat.: right --> 400,0.4250; wrong --> 600,0.0000
3. Rule 3, with a relevance of 0.370 (based on 400 examples) and an error of 0.000 (based on 600 examples)
   Outcome "0", no. of conditions --> 2
   Condition 1 (relevance 0.810000): R4 <= 11.6000
   Condition 2 (relevance 0.050000): R7 > 6.4500
   Stat.: right --> 400,0.3700; wrong --> 600,0.0000
4. Rule 4, with a relevance of 0.355 (based on 400 examples) and an error of 0.000 (based on 600 examples)
   Outcome "0", no. of conditions --> 2
   Condition 1 (relevance 0.480000): R4 <= 14.3250
   Condition 2 (relevance 0.235000): R7 > 8.7600
   Stat.: right --> 400,0.3550; wrong --> 600,0.0000
5. Rule 5, with a relevance of 0.340 (based on 400 examples) and an error of 0.000 (based on 600 examples)
   Outcome "0", no. of conditions --> 2
   Condition 1 (relevance 0.900000): R4 <= 10.3600
   Condition 2 (relevance 0.020000): R7 > 4.8250
   Stat.: right --> 400,0.3400; wrong --> 600,0.0000
6. Rule 6, with a relevance of 0.305 (based on 400 examples) and an error of 0.000 (based on 600 examples)
   Outcome "0", no. of conditions --> 3
   Condition 1 (relevance 0.551667): R4 <= 13.6350
   Condition 2 (relevance 0.018333): R7 > 7.7150
   Condition 3 (relevance 0.005000): R10 > 21.9950
   Stat.: right --> 400,0.3050; wrong --> 600,0.0000
7. Rule 7, with a relevance of 0.265 (based on 400 examples) and an error of 0.000 (based on 600 examples)
   Outcome "0", no. of conditions --> 3
   Condition 1 (relevance 0.415000): R4 <= 14.8650
   Condition 2 (relevance 0.038333): R7 > 8.7600
   Condition 3 (relevance 0.006667): R10 > 23.7300
   Stat.: right --> 400,0.2650; wrong --> 600,0.0000
8. Rule 8, with a relevance of 0.235 (based on 400 examples) and an error of 0.000 (based on 600 examples)
   Outcome "0", no. of conditions --> 2
   Condition 1 (relevance 0.320000): R4 <= 14.8650
   Condition 2 (relevance 0.288333): R7 > 9.6750
   Stat.: right --> 400,0.2350; wrong --> 600,0.0000
9. Rule 9, with a relevance of 0.230 (based on 400 examples) and an error of 0.000 (based on 600 examples)
   Outcome "0", no. of conditions --> 2
   Condition 1 (relevance 0.960000): R4 <= 8.7400
   Condition 2 (relevance 0.003333): R7 > 3.6100
   Stat.: right --> 400,0.2300; wrong --> 600,0.0000
10. Rule 10, with a relevance of 0.218 (based on 400 examples) and an error of 0.000 (based on 600 examples)
   Outcome "0", no. of conditions --> 2
   Condition 1 (relevance 0.240000): R4 <= 15.8450
   Condition 2 (relevance 0.415000): R7 > 10.0100
   Stat.: right --> 400,0.2175; wrong --> 600,0.0000
11. Rule 11, with a relevance of 0.185 (based on 400 examples) and an error of 0.000 (based on 600 examples)
   Outcome "0", no. of conditions --> 2
   Condition 1 (relevance 0.623333): R4 <= 11.6000
   Condition 2 (relevance 0.050000): R10 > 21.9950
   Stat.: right --> 400,0.1850; wrong --> 600,0.0000
12. Rule 12, with a relevance of 0.170 (based on 400 examples) and an error of 0.000 (based on 600 examples)
   Outcome "0", no. of conditions --> 3
   Condition 1 (relevance 0.203333): R4 <= 13.3400
   Condition 2 (relevance 0.006667): R7 > 6.7250
   Condition 3 (relevance 0.016667): 23.7300 < R10 <= 27.5700
   Stat.: right --> 400,0.1700; wrong --> 600,0.0000
13. Rule 13, with a relevance of 0.160 (based on 400 examples) and an error of 0.000 (based on 600 examples)
   Outcome "0", no. of conditions --> 3
   Condition 1 (relevance 0.898333): R4 <= 10.9550
   Condition 2 (relevance 0.001667): R6 > 4.8500
   Condition 3 (relevance 0.008333): R7 > 4.8250
   Stat.: right --> 400,0.1600; wrong --> 600,0.0000
14. Rule 14, with a relevance of 0.135 (based on 400 examples) and an error of 0.000 (based on 600 examples)
   Outcome "0", no. of conditions --> 3
   Condition 1 (relevance 0.958333): R4 <= 9.5200
   Condition 2 (relevance 0.001667): R6 > 4.2000
   Condition 3 (relevance 0.001667): R7 > 3.6100
   Stat.: right --> 400,0.1350; wrong --> 600,0.0000
15. Rule 15, with a relevance of 0.125 (based on 400 examples) and an error of 0.000 (based on 600 examples)
   Outcome "0", no. of conditions --> 1
   Condition 1 (relevance 1.000000): R4 <= 6.9050
   Stat.: right --> 400,0.1250; wrong --> 600,0.0000
16. Rule 16, with a relevance of 0.125 (based on 400 examples) and an error of 0.000 (based on 600 examples)
   Outcome "0", no. of conditions --> 3
   Condition 1 (relevance 0.943333): R4 <= 10.3600
   Condition 2 (relevance 0.005000): R6 > 4.8500
   Condition 3 (relevance 0.001667): R7 > 3.6100
   Stat.: right --> 400,0.1250; wrong --> 600,0.0000
17. Rule 17, with a relevance of 0.102 (based on 400 examples) and an error of 0.000 (based on 600 examples)
   Outcome "0", no. of conditions --> 3
   Condition 1 (relevance 0.298333): R4 <= 14.3250
   Condition 2 (relevance 0.006667): R7 > 7.7150
   Condition 3 (relevance 0.028333): R10 > 27.5700
   Stat.: right --> 400,0.1025; wrong --> 600,0.0000
18. Rule 18, with a relevance of 0.078 (based on 400 examples) and an error of 0.000 (based on 600 examples)
   Outcome "0", no. of conditions --> 3
   Condition 1 (relevance 0.098333): R4 <= 16.8900
   Condition 2 (relevance 0.068333): R7 > 10.7850
   Condition 3 (relevance 0.001667): R10 > 27.5700
   Stat.: right --> 400,0.0775; wrong --> 600,0.0000
19. Rule 19, with a relevance of 0.075 (based on 400 examples) and an error of 0.000 (based on 600 examples)
   Outcome "0", no. of conditions --> 3
   Condition 1 (relevance 0.176667): R4 <= 15.8450
   Condition 2 (relevance 0.015000): R7 > 9.6750
   Condition 3 (relevance 0.010000): R10 > 28.7550
   Stat.: right --> 400,0.0750; wrong --> 600,0.0000
20. Rule 20, with a relevance of 0.065 (based on 400 examples) and an error of 0.000 (based on 600 examples)
   Outcome "0", no. of conditions --> 2
   Condition 1 (relevance 0.988333): R4 <= 8.7400
   Condition 2 (relevance 0.003333): R6 > 4.2000
   Stat.: right --> 400,0.0650; wrong --> 600,0.0000
21. Rule 21, with a relevance 0.058 (based on 400 examples) and an error of 0.000 (based on 600 examples)
   Outcome "0", no. of conditions --> 2
   Condition 1 (relevance 0.966667): R4 <= 9.5200
   Condition 2 (relevance 0.010000): R6 > 4.8500
   Stat.: right --> 400,0.0575; wrong --> 600,0.0000
22. Rule 22, with a relevance 0.485 (based on 600 examples) and an error of 0.000 (based on 400 examples)
   Outcome "1", no. of conditions --> 2
   Condition 1 (relevance 0.900000): R4 > 15.8450
   Condition 2 (relevance 0.022500): R7 <= 10.7850
   Stat.: right --> 600,0.4850; wrong --> 400,0.0000
23. Rule 23, with a relevance 0.430 (based on 600 examples) and an error of 0.000 (based on 400 examples)
   Outcome "1", no. of conditions --> 1
   Condition 1 (relevance 1.000000): R4 > 16.8900
   Stat.: right --> 600,0.4300; wrong --> 400,0.0000
24. Rule 24, with a relevance of 0.393 (based on 600 examples) and an error of 0.000 (based on 400 examples)
   Outcome "1", no. of conditions --> 3
   Condition 1 (relevance 0.725000): R4 > 14.8650
   Condition 2 (relevance 0.032500): R7 <= 10.0100
   Condition 3 (relevance 0.005000): R10 <= 28.7550
   Stat.: right --> 600,0.3933; wrong --> 400,0.0000
25. Rule 25, with a relevance of 0.392 (based on 600 examples) and an error of 0.000 (based on 400 examples)
   Outcome "1", no. of conditions --> 2
   Condition 1 (relevance 0.680000): R4 > 14.8650
   Condition 2 (relevance 0.085000): R7 <= 9.6750
   Stat.: right --> 600,0.3917; wrong --> 400,0.0000
26. Rule 26, with a relevance of 0.310 (based on 600 examples) and an error of 0.000 (based on 400 examples)
   Outcome "1", no. of conditions --> 3
   Condition 1 (relevance 0.497500): R4 > 13.6350
   Condition 2 (relevance 0.077500): R7 <= 8.7600
   Condition 3 (relevance 0.005000): R10 <= 27.5700
   Stat.: right --> 600,0.3100; wrong --> 400,0.0000
27. Rule 27, with a relevance of 0.297 (based on 600 examples) and an error of 0.000 (based on 400 examples)
   Outcome "1", no. of conditions --> 2
   Condition 1 (relevance 0.805000): R4 > 15.8450
   Condition 2 (relevance 0.022500): R10 <= 27.5700
   Stat.: right --> 600,0.2967; wrong --> 400,0.0000
28. Rule 28, with a relevance of 0.285 (based on 600 examples) and an error of 0.000 (based on 400 examples)
   Outcome "1", no. of conditions --> 2
   Condition 1 (relevance 0.520000): R4 > 14.3250
   Condition 2 (relevance 0.125000): R7 <= 8.7600
   Stat.: right --> 600,0.2850; wrong --> 400,0.0000
29. Rule 29, with a relevance of 0.233 (based on 600 examples) and an error of 0.000 (based on 400 examples)
   Outcome "1", no. of conditions --> 3
   Condition 1 (relevance 0.505000): R4 > 14.3250
   Condition 2 (relevance 0.007500): R7 <= 9.6750
   Condition 3 (relevance 0.010000): R10 <= 23.7300
   Stat.: right --> 600,0.2333; wrong --> 400,0.0000
30. Rule 30, with a relevance of 0.220 (based on 600 examples) and an error of 0.000 (based on 400 examples)
   Outcome "1", no. of conditions --> 3
   Condition 1 (relevance 0.380000): R4 > 13.3400
   Condition 2 (relevance 0.015000): R7 <= 8.7600
   Condition 3 (relevance 0.012500): R10 <= 21.9950
   Stat.: right --> 600,0.2200; wrong --> 400,0.0000
31. Rule 31, with a relevance of 0.190 (based on 600 examples) and an error of 0.000 (based on 400 examples)
   Outcome "1", no. of conditions --> 3
   Condition 1 (relevance 0.282500): R4 > 12.2950
   Condition 2 (relevance 0.087500): R7 <= 7.7150
   Condition 3 (relevance 0.002500): R10 <= 23.7300
   Stat.: right --> 600,0.1900; wrong --> 400,0.0000
32. Rule 32, with a relevance of 0.178 (based on 600 examples) and an error of 0.000 (based on 400 examples)
   Outcome "1", no. of conditions --> 2
   Condition 1 (relevance 0.320000): R4 > 13.3400
   Condition 2 (relevance 0.232500): R7 <= 7.7150
   Stat.: right --> 600,0.1783; wrong --> 400,0.0000
33. Rule 33, with a relevance of 0.160 (based on 600 examples) and an error of 0.000 (based on 400 examples)
   Outcome "1", no. of conditions --> 2
   Condition 1 (relevance 0.210000): R4 > 11.6000
   Condition 2 (relevance 0.440000): R7 <= 6.7250
   Stat.: right --> 600,0.1600; wrong --> 400,0.0000
34. Rule 34, with a relevance of 0.142 (based on 600 examples) and an error of 0.000 (based on 400 examples)
   Outcome "1", no. of conditions --> 3
   Condition 1 (relevance 0.182500): R4 > 10.9550
   Condition 2 (relevance 0.170000): R7 <= 6.4500
   Condition 3 (relevance 0.002500): R10 <= 23.7300
   Stat.: right --> 600,0.1417; wrong --> 400,0.0000
35. Rule 35, with a relevance of 0.080 (based on 600 examples) and an error of 0.000 (based on 400 examples)
   Outcome "1", no. of conditions --> 2
   Condition 1 (relevance 0.100000): R4 > 10.3600
   Condition 2 (relevance 0.560000): R7 <= 4.8250
   Stat.: right --> 600,0.0800; wrong --> 400,0.0000
36. Rule 36, with a relevance of 0.032 (based on 600 examples) and an error of 0.000 (based on 400 examples)
   Outcome "1", no. of conditions --> 2
   Condition 1 (relevance 0.040000): R4 > 9.5200
   Condition 2 (relevance 0.647500): R7 <= 3.6100
   Stat.: right --> 600,0.0317; wrong --> 400,0.0000
37. Rule 37, with a relevance of 0.022 (based on 600 examples) and an error of 0.000 (based on 400 examples)
   Outcome "1", no. of conditions --> 3
   Condition 1 (relevance 0.077500): R4 > 9.5200
   Condition 2 (relevance 0.007500): R6 <= 4.8500
   Condition 3 (relevance 0.017500): R7 <= 4.8250
   Stat.: right --> 600,0.0217; wrong --> 400,0.0000
38. Rule 38, with a relevance of 0.020 (based on 600 examples) and an error of 0.000 (based on 400 examples)
   Outcome "1", no. of conditions --> 3
   Condition 1 (relevance 0.007500): R4 > 12.2950
   Condition 2 (relevance 0.162500): R7 <= 7.7150
   Condition 3 (relevance 0.002500): R10 > 27.5700
   Stat.: right --> 600,0.0200; wrong --> 400,0.0000
39. Rule 39, with a relevance of 0.015 (based on 600 examples) and an error of 0.000 (based on 400 examples)
   Outcome "1", no. of conditions --> 2
   Condition 1 (relevance 0.312500): R4 > 10.3600
   Condition 2 (relevance 0.560000): R6 <= 4.8500
   Stat.: right --> 600,0.0150; wrong --> 400,0.0000
40. Rule 40, with a relevance of 0.013 (based on 600 examples) and an error of 0.000 (based on 400 examples)
   Outcome "1", no. of conditions --> 3
   Condition 1 (relevance 0.037500): R4 > 8.7400
   Condition 2 (relevance 0.002500): R6 <= 4.8500
   Condition 3 (relevance 0.062500): R7 <= 3.6100
   Stat.: right --> 600,0.0133; wrong --> 400,0.0000
41. Rule 41, with a relevance of 0.010 (based on 600 examples) and an error of 0.000 (based on 400 examples)
   Outcome "1", no. of conditions --> 3
   Condition 1 (relevance 0.025000): R4 > 6.9050
   Condition 2 (relevance 0.012500): R6 <= 4.2000
   Condition 3 (relevance 0.082500): R7 <= 3.6100
   Stat.: right --> 600,0.0100; wrong --> 400,0.0000
42. Rule 42, with a relevance of 0.008 (based on 600 examples) and an error of 0.000 (based on 400 examples)
   Outcome "1", no. of conditions --> 2
   Condition 1 (relevance 0.202500): R4 > 8.7400
   Condition 2 (relevance 0.732500): R6 <= 4.2000
   Stat.: right --> 600,0.0083; wrong --> 400,0.0000

In geometrical terms, the identified rules represent a constant segmented approximation (since the rules are threshold rules) of the hypersurface which, in the sensor response space, discriminates between the outcome "1" and the outcome "0".

We were able to configure a microcontroller of the Boolean learning machine on the basis of the collection of binary classification rules determined in this way.

The machine configured in this way is capable of classifying a response vector generated by the sensor array by application of the predetermined collection of rules, using the following procedure:
- firstly, the rules describing a first outcome, for example the outcome "0", are applied, and, if at least one of these describes the response vector in question, the one having the highest relevance is chosen;
- secondly, the rules describing the outcome "1" are applied, and, if at least one of these describes the response vector in question, the one having the highest relevance is chosen;
- finally, the rule having the higher relevance of these two is considered to be the rule capable of classifying the measurement in question.

It should be noted that the rules found in this way are not only adapted to classify new response vectors different from the training vectors, but provide also some other very useful information, namely the number and identities of the sensors in the array which supply the greatest information for the detection of the substance and/or mixture in question.

In the case described by way of example, it can be established from the rules listed above that:
i) the sensor identified by the number 4 is present in the largest number of rules, and in particular has a total relevance of 98.8%;
ii) the sensor identified by the number 7 has a relevance of 46.8%;
iii) the sensor identified by the number 10 has a relevance of 4.5%;
iv) the sensor identified by the number 6 has a relevance of 1.9%; and
v) the sensors identified by the numbers 8 and 9 do not carry information for discriminating ethanol in a concentration of more or less than 30 ppm.

The example which has been described relates to a method for discriminating between a pair of predetermined complementary outcomes of the detection, for example outcomes representing the presence or absence of a predetermined chemical substance or representing the comparison of the concentration of the said substance with a predetermined threshold value.

If we wish to introduce a concept of measurement, it is essential to discretize the information which is to be obtained, for example by generating from a training response vector a plurality of collections of rules which describe the outcome representing a concentration of ethanol above a first threshold value, for example 5 ppm, that representing a concentration of ethanol above a second threshold value, for example 15 ppm, that representing a concentration of ethanol above a third threshold value, for example 25 ppm, and so on.

These collections of rules can then be used to classify a new response vector in one of the predetermined ranges of concentration of ethanol, and thus, substantially, to provide a measurement of concentration. Clearly, an increase in the desired precision of the measurement will require more collections of rules to enable the learning machine to resolve the problem, in other words more comparison threshold values.

In general, a plurality of collections of rules is adapted to discriminate among a plurality of predetermined outcomes of the detection, representing corresponding concentrations of at least one predetermined chemical substance in a mixture of substances, in order to provide a classification indicating a quantitative measurement of the concentration of the said substance.

Advantageously, the communication interface module 36 permits the acquisition of the collection(s) of rules from remote devices, thus enabling the classification rules to be programmed as desired, in order to provide a reconfigurable sensor arrangement whose characteristics can be varied simply by changing the rules by which it operates, but leaving the sensor array unchanged.

Similarly, it is possible to provide a sensor arrangement selective for a plurality of substances in parallel, by generating a plurality of collections of rules which are applied successively by the learning machine according to a predetermined criterion, in such a way that it is possible to detect the presence and concentration of a plurality of substances present simultaneously in an environment.

Advantageously, the modification of the collection of rules of the learning machine is useful in all cases in which the conditions of use of the sensor arrangement can vary, for example as a result of installation in different environments or exposure to different substances, but also for the purpose of allowing for the ageing of the sensors and possible drifts of their physical and chemical properties.

Advantageously, since the rules required for the operation of the learning machine can be encoded in Boolean form and essentially require the execution of AND and OR operations, it is possible to use elementary logic circuits or processors having a modest computing capacity for their implementation, thus permitting the production of sensor arrangements which are inexpensive, but equally reliable and precise.

The fundamental principle of the invention is intended to be applied to any type of sensor. Advantageously, it is possible to integrate the sensors by means of techniques of the PMCS type, consisting of the deposition of nano-clusters in a controlled way to produce nanostructured films, in order to permit the efficient control of the structure of the sensitive films together with the integration of the sensor array and the logic circuit of the learning machine in silicon-based planar technology.

Clearly, provided that the principle of the invention is retained, the forms of embodiment and in particular the forms of construction can be varied widely from what has been described and illustrated purely by way of example and without restrictive intent, without departure from the scope of protection of the present invention as defined by the attached claims.

## Claims

1. Sensor arrangement of the electronic nose type for the qualitative and quantitative detection of chemical substances and/or mixtures of substances in an environment, comprising:
- an array of sensors (10), each of which is capable of modifying at least one of its own physical parameters with respect to a reference condition in the presence of at least one predetermined chemical substance, and emitting a corresponding electrical response signal indicating the extent of modification of the said parameter,
the set of the response signals of the sensor array (10) being correlated with the presence and/or concentration of the said at least one substance; and
- electronic processing and recognition means of the automatic learning type (30), arranged to receive the said response signals at their input and to detect the presence and/or concentration of at least one chemical substance being searched for, on the basis of a set of training data acquired in a learning phase,
**characterized in that** the said processing and recognition means (30) include a Boolean learning machine (34), arranged to carry out the classification of the set of response signals generated by the sensor array (10) by the application of at least one predetermined collection of binary classification rules, which is adapted to discriminate an outcome from a pair of predetermined complementary outcomes of the detection.

2. Sensor arrangement according to Claim 1, in which the said Boolean learning machine (34) is arranged to carry out the classification of the set of response signals generated by the sensor array (10) by the application of a plurality of collections of binary classification rules, which is adapted to discriminate among a plurality of predetermined outcomes of the detection.

3. Sensor arrangement according to any one of the preceding claims, in which the said collection of rules is obtainable by the said machine in a learning phase on the basis of the said set of training data by a method for generating Boolean rules based on the application of the Hamming Clustering algorithm.

4. Sensor arrangement according to Claim 3, in which the said set of training data includes a plurality of sets of response signals of the sensor array (10) representing the presence and/or concentration of known chemical substances and/or mixtures of substances.

5. Sensor arrangement according to Claim 4, in which the said learning machine (34) is arranged to obtain the said collection of binary classification rules as a function of a subset of the response signals of the sensor array (10).

6. Sensor arrangement according to any one of the preceding claims, in which the said learning machine (34) comprises a programmable logic device is adapted to apply to each set of response signals a predetermined collection of binary classification rules of the "if-then" type, corresponding to the expression of a two-level Boolean function in the form of a sum of logical products of binary variables indicative of the comparison between each response signal and a corresponding predetermined threshold value.

7. Sensor arrangement according to any one of the preceding claims, in which the said learning machine (34) comprises a set of logic gates configured as a combinatorial logic network.

8. Sensor arrangement according to Claim 7, in which the said set of logic gates is a set of logic gates with a variable configuration.

9. Sensor arrangement according to any one of Claims 1 to 6, in which the said learning machine (34) comprises a programmable logic device adapted to apply to the set of response signals a predetermined sequence of collections of rules, each collection of rules being adapted to detect a different chemical substance and/or mixture of substances.

10. Sensor arrangement according to any one of the preceding claims, in which the said processing and recognition means (30) are associated with interface means (36) for acquiring the said collection of rules from remote devices.

11. Sensor arrangement according to any one of the preceding claims, in which the said processing and recognition means (30) include a module (32) for extracting the characteristics of the response signals emitted by the sensors of the said array (10), adapted to acquire the value of each signal in a transient phase of variation of a physical parameter of the sensor, and to estimate a steady-state value of the signal indicating the modification of the said parameter according to predetermined rules.

12. Sensor arrangement according to any one of the preceding claims, in which the said physical parameter is the resistive characteristic of a sensitive film sensor.

13. Method for the qualitative and quantitative detection of chemical substances and/or mixtures of substances in an environment by means of a sensor arrangement of the electronic nose type, comprising:
- an array of sensors (10), each of which is capable of modifying at least one of its own physical parameters with respect to a reference condition in the presence of at least one predetermined chemical substance, and emitting a corresponding electrical response signal indicating the modification of the said parameter,
the set of the response signals of the sensor array being correlated with the presence and/or concentration of the said at least one substance; and
- electronic processing and recognition means (30) including a Boolean learning machine (34), arranged to receive the said response signals at their input and to detect the presence and/or concentration of at least one chemical substance being searched for, on the basis of a set of training data,
the method being **characterized in that** it comprises the operations of:
- generating, in a learning phase and on the basis of a plurality of sets of training response signals, at least one predetermined collection of binary classification rules, which is adapted to perform a discrimination in a pair of predetermined complementary outcomes of the detection;
- configuring the processing and recognition means (30) in accordance with the said collection of rules; and
- classifying a set of response signals generated by the sensor array (10), by application of the said at least one predetermined collection of rules.

14. Method according to Claim 13, in which the set of response signals generated by the sensor array (10) is classified by the application of a plurality of collections of rules, which is adapted to discriminate among a plurality of predetermined outcomes of the detection.

15. Method according to Claim 13 or 14, in which the classification of a set of response signals includes:
- the application of a first set of rules of a collection, adapted to represent a first predetermined outcome of the detection;
- the application of a second set of rules of the said collection, adapted to represent a second predetermined outcome of the detection;
- the application, if appropriate, of further sets of rules of other collections, adapted to represent corresponding further predetermined outcomes of the detection; and
- the classification of the set of response signals according to the outcome represented by the rule having the greatest relevance, in other words that is adapted to correctly represent the outcome of the detection for the greatest number of sets of training response signals.

16. Method according to Claim 13 or 14, in which the generation of a collection of rules includes the operations of:
- encoding each training response signal of the sensor array (10) as a corresponding binary string by application of a predetermined transformation function capable of preserving sequencing and distance properties of the values which each signal can take;
- concatenating the binary strings generated for each signal of a set of response signals of the sensor array (10) and associating these with an outcome of the detection; and
- synthesizing the expression of a Boolean function with AND/OR operators of the encoded signals, adapted to discriminate between a pair of predetermined outcomes of the detection.

17. Method according to Claim 16, in which the encoding of each response signal as a binary string includes the discretization of the value of the signal and the association of a binary string with each discrete value according to a "thermometer encoding".

18. Method according to Claim 16, in which the expression of the Boolean function is synthesized by application of the Hamming Clustering algorithm, and includes the aggregation of binary strings which are associated with a single outcome of the detection and which are close in terms of the Hamming distance, so as to generate prime implicants of a Boolean function expression in the form of a sum of logical products.

19. Method according to Claim 18, including the translation of each logical product of the Boolean expression into an intelligible rule of the "if-then" type.

20. Method according to Claim 16, including the application of a logic network synthesis technique for determining the configuration of a programmable logic device as the Boolean learning machine (34).

21. Method according to Claim 20, in which the configuration of the programmable logic device takes place by means of a programming operation carried out by remote devices.
